# EUROPÄISCHE PATENTSCHRIFT

(11) **EP 2 682 086 B1**
(45) Veröffentlichungstag und Bekanntmachung des Hinweises auf die Patenterteilung: **28.10.2015**
(21) Anmeldenummer: 13003311.1
(22) Anmeldetag: 28.06.2013
(51) Int. Cl.: A61G 15/08, A61G 13/12, A61B 19/00

(54) **Mobile Behandlungsstation zum Einsatz im Gesundheitswesen**
Mobile treatment station for use in the health sector
Station de traitement mobile pour une utilisation dans des services de santé

(30) Priorität: 06.07.2012 DE 102012013610
(43) Veröffentlichungstag der Anmeldung: 08.01.2014
(73) Patentinhaber: Majstorovic, Branka, 45127 Essen (DE)
(72) Erfinder: Majstorovic, Branka, 45127 Essen (DE)
(74) Vertreter: Schleitzer, Dirk-Karsten

(56) Entgegenhaltungen:
- DE-A1- 19 926 062
- GB-A- 334 209
- GB-A- 466 899
- GB-A- 2 274 244

## Beschreibung

Die Erfindung bezieht sich auf eine mobile Behandlungsstation zum Einsatz im Gesundheitswesen, z.B. in Krankenhäusern, Institutionen der Altenpflege, Rehabilitationszentren ud.dgl.

In den genannten Institutionen tritt häufig der Fall auf, dass eine behandelnde Person, z.B. eine Krankenschwester, ein Arzt, ein Altenpfleger od.dgl., eine zu behandelnde Person, z.B. einen Patienten, behandeln bzw. versorgen muss, wobei sich die zu behandelnde Person während der Versorgung bzw. während der Behandlung weiterhin in ihrem Krankenbett befindet.

Bei der Durchführung derartiger Behandlungs- und/oder Versorgungsmaßnahmen kommt es häufig dazu, dass die behandelnde Person eine vergleichsweise ungünstige und ergonomisch schädliche Körperhaltung einnehmen muss. Darüber hinaus sind für solche Behandlungs- und/oder Versorgungsmaßnahmen häufig mehr als eine zu behandelnde Person erforderlich, da eine Gliedmaße oder ein Körperteil der zu behandelnden Person in einer bestimmten Position fixiert werden muss. Dies ist beispielsweise bei dem Wechseln eines Verbandes oder Reinigungsmaßnahmen der Fall Dokument GB334209 offenbart eine mobile Behandlungsstation mit allen technischen Merkmalen des Oberbegriffs des Anspruchs 1.

Der Erfindung liegt die Aufgabe zugrunde, die Durchführung derartiger Versorgungs- und/oder Behandlungsmaßnahmen erheblich zu vereinfachen, wobei darüber hinaus ungünstige und schädliche Körperhaltungen der zu behandelnden Personen ausgeschlossen werden sollen.

Diese Aufgabe wird erfindungsgemäß durch eine mobile Behandlungsstation zum Einsatz im Gesundheitswesen, z.B. in Krankenhäusern, Institutionen der Altenpflege, Rehabilitationszentren ud.dgl., nach Anspruch 1, gelöst, wobei zu dieser mobilen Behandlungsstation ein Sitzteil, auf dem eine zu behandelnde Person sitzen kann, ein Stützteil, auf dem eine Gliedmaße oder ein Körperteil einer zu behandelnden Person abstützbar ist, und ein mechanisches Verbindungsglied gehören, mittels dem das Sitzteil und das Stützteil fest miteinander verbindbar sind. Die erfindungsgemäße mobile Behandlungsstation bietet für die behandelnde Person, d.h. für die Krankenschwester, den Arzt, den Altenpfleger od.dgl., eine Sitzmöglichkeit. Des Weiteren kann mittels der erfindungsgemäßen mobilen Behandlungsstation das zu behandelnde Körperteil abgestützt werden, und zwar in einer Position, in der es für die sitzende behandelnde Person in einfacher Weise bearbeitbar ist. Die erfindungsgemäße mobile Behandlungsstation kann mittels einer einzigen Hand bequem transportiert bzw. gezogen werden, da das Sitzteil und das Stützteil mittels des mechanischen Verbindungsglieds fest miteinander verbunden sind. Durch den Einsatz der erfindungsgemäßen mobilen Behandlungsstation können viele Behandlungs- und Versorgungsmaßnahmen von einer einzigen behandelnden Person durchgeführt werden. Insbesondere kann auf eine zusätzliche behandelnde Person verzichtet werden, wenn diese lediglich zum Stützen des zu behandelnden Körperteils eingesetzt werden soll. Dies ist z.B. der Fall, wenn Verbände, Kompressionsverbände od.dgl. zu wechseln sind.

Da das zu behandelnde Körperteil bei der Behandlung bzw. der Versorgung auf dem Stützteil und nicht im Bett angeordnet ist, lassen sich Versorgungs- und Behandlungsmaßnahmen, z.B. Verbandswechsel, hygienischer durchführen. Die erfindungsgemäße mobile Behandlungsstation ermöglicht insgesamt ein schnelleres, hygienischeres und bequemeres Arbeiten an der zu behandelnden Person bzw. am Patienten. Hierdurch lassen sich Qualitäts- und Zeitvorteile bei der Durchführung der geschilderten Behandlungs- und Versorgungsmaßnahmen erreichen. Die vorstehend geschilderte mobile Behandlungsstation kann mit einem vergleichsweise geringen Aufwand gereinigt und desinfiziert werden, wobei sämtliche hygienischen Standards ohne weiteres erfüllbar sind.

Gemäß einer vorteilhaften Weiterbildung der erfindungsgemäßen mobilen Behandlungsstation weist deren Sitzteil ein höhenverstellbares Sitzelement auf. Durch die individuell verstellbare vertikale Position dieses Sitzelements kann die Sitzhöhe an die Körperstatur der behandelnden Person angepasst werden.

Das mechanische Verbindungsglied der erfindungsgemäßen mobilen Behandlungsstation ist längenverstellbar, und als Teleskopstange ausgebildet, so dass für das Sitzelement des Sitzteils eine in Bezug auf das Stützteil optimale Position eingestellt werden kann, bei der sowohl die vertikale Stellung als auch die horizontale Stellung des Sitzelements optimal anpasssbar ist. Hierdurch ergibt sich für die behandelnde Person eine rückenschonende Arbeitsweise. Aufgrund der optimalen Sitzposition der behandelnden Person werden ggf. vorhandene Rückenbeschwerden gelindert. Ein schmerzfreies Arbeiten ist möglich. Darüber hinaus wird das Auftreten von Rückenbeschwerden prophylaktisch reduziert, wodurch geringere krankheitsbedingte Ausfallzeiten des Personals von Institutionen des Gesundheitswesens auftreten.

Gemäß einer weiteren vorteilhaften Ausführungsform der erfindungsgemäßen mobilen Behandlungsstation kann es vorgesehen sein, auch am Stützteil eine höhenverstellbare Auflagefläche vorzusehen. Hierdurch ist es möglich, die Vertikalposition der Auflagefläche des Stützteils an unterschiedliche Bettenhöhen anzugleichen.

Vorteilhaft ist am Stützteil der erfindungsgemäßen mobilen Behandlungsstation ein Aufnahmekorb vorgesehen, der der Aufnahme von Behandlungsutensilien dient. Dieser Aufnahmekorb ist vorzugsweise verstellbar am Stützteil angebracht. Zusätzlich zum Aufnahmekorb kann auch eine Ablagefläche od.dgl. am Stützteil angebracht sein. Hierdurch wird die Durchführung von Behandlungs- und/oder Versorgungsmaßnahmen weiter erleichtert.

Zum einfachen und mit einer Hand ohne großen Aufwand durchführbaren Transport der erfindungsgemäßen mobilen Behandlungsstation sind deren Stützteil und deren Sitzteil auf Rollen verfahrbar, wobei am Stützteil und/oder am Sitzteil eine Bremsvorrichtung vorgesehen ist. Hierdurch kann die mobile Behandlungsstation mit einem geringen Aufwand örtlich fixiert werden.

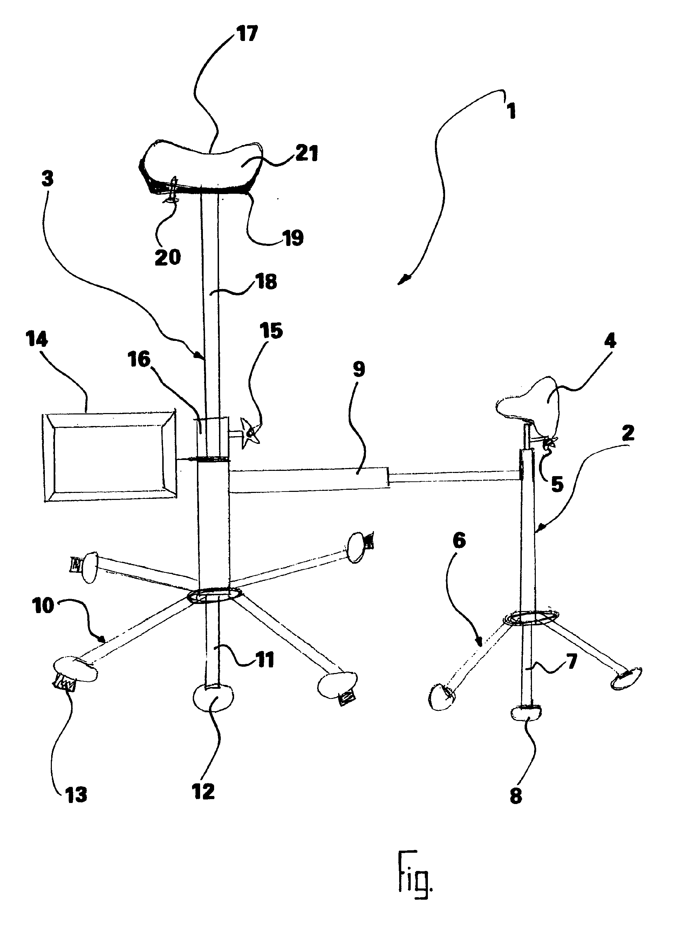

Im Folgenden wird die Erfindung anhand einer Ausführungsform unter Bezugnahme auf die Zeichnung näher erläutert, in deren einziger Figur ein Ausführungsbeispiel einer erfindungsgemäßen mobilen Behandlungsstation zum Einsatz im Gesundheitswesen prinzipiell dargestellt ist.

Eine in der Figur gezeigte Ausführungsform einer erfindungsgemäßen mobilen Behandlungsstation 1 hat ein Sitzteil 2 und ein Stützteil 3.

Das Sitzteil 2 dient dazu, für eine behandelnde Person eine Sitzmöglichkeit zu schaffen, die der behandelnden Person die Möglichkeit bietet, in einer bequemen Körperstellung eine zu behandelnde Person zu behandeln. Hierzu weist das Sitzteil ein Sitzelement 4 auf, welches in Vertikalrichtung des Sitzteils verstellbar ist. Dieses Sitzteil kann mittels einer Feststellschraube 5 in verschiedenen Vertikalpositionen am Sitzteil 2 fixiert werden. Des Weiteren ist das Sitzteil mit einem Fahrgestell 6 mit mehreren Rollenträgern 7 versehen, an deren freien Enden Rollen 8 drehbar gelagert sind.

Das Sitzteil 2 ist mittels eines mechanischen Verbindungsglieds 9, das im dargestellten Ausführungsbeispiel als in ihrer Länge veränderbare Teleskopstange 9 ausgebildet ist, mit dem Stützteil 3 fest verbunden. Die Teleskopstange 9 ist in beliebiger Länge fixierbar, so dass der horizontale Abstand zwischen dem Sitzteil 2 einerseits und dem Stützteil 3 andererseits beliebig einstellbar ist.

An seinem unteren Ende ist das Stützteil 3 ebenfalls mit einem Fahrgestell 10 versehen, dessen Rollenträger 11 an ihren freien Enden mit Rollen 12, die drehbar dort gelagert sind, ausgerüstet ist. An einer oder mehreren der Rollen 12 des Fahrgestells 10 des Stützteils 3 ist im dargestellten Ausführungsbeispiel der erfindungsgemäßen mobilen Behandlungsstation eine Bremsvorrichtung 13 angeordnet. Mittels der Bremsvorrichtung 13 ist das Stützteil 3 und damit in gewisser Weise auch das Sitzteil 2 bremsbar.

Oberhalb des Fahrgestells 10 und des stützteilseitigen Endes der Teleskopstange 9 ist am Stützteil 3 ein Aufnahmekorb 14 angeordnet. Dieser Aufnahmekorb 14 dient der Aufnahme unterschiedlichster Behandlungsutensilien, die von der behandelnden Person benötigt werden. Der Aufnahmekorb 14 ist mittels einer Feststellschraube 15, die einer buchsenförmigen Halterung 16 des Aufnahmekorbs 14 zugeordnet ist, in unterschiedlichen Vertikal- und Drehpositionen am Stützteil 3 fixierbar.

An seinem oberen Ende weist das Stützteil 3 eine Auflagefläche 17 auf, die im dargestellten Ausführungsbeispiel aus einer an das obere Ende einer Vertikalstange 18 des Stützteils 3 angeschweißten Metallplatte 19 und einem an der Oberseite dieser Metallplatte 19 mittels Befestigungsschrauben 20 angebrachten Schaumpolsterelement 21 besteht. Das Schaumpolsterelement 21 kann z.B. mit PVC ummantelt sein.

Auf der Auflagefläche 17 kann z.B. ein Fuß, ein Knie, ein Ellbogen oder ein anderes Körperteil gehaltert werden, welches von der auf dem Sitzelement 4 des Sitzteils 2 sitzenden behandelnden Person zu bearbeiten ist. Hierbei kann die behandelnde Person nicht nur die Vertikalposition des Sitzelements entsprechend ihrer Körperstatur einstellen, sondern sie kann auch - je nach Art der vorzunehmenden Behandlung - den Horizontalabstand zwischen Stützteil 3 und Sitzteil 2 beliebig einstellen. Auch bei dieser horizontalen Einstellung können selbstverständlich körperliche Merkmale der behandelnden Person berücksichtigt werden.

## Patentansprüche

1. Mobile Behandlungsstation zum Einsatz im Gesundheitswesen, z.B. in Krankenhäusern, Institutionen der Altenpflege, Rehabilitationszentren ud.dgl., mit einem Sitzteil (2), auf dem eine behandelnde Person sitzen kann, einem Stützteil (3), auf dem eine Gliedmaße einer zu behandelnden Person abstützbar ist, und einem mechanischen Verbindungsglied (9), mittels dem das Sitzteil (2) und das Stützteil (3) miteinander verbunden sind, **dadurch gekennzeichnet, dass** das mechanische Verbindungsglied (9) längenverstellbar und als Teleskopstange (9) ausgebildet ist, so dass der horizontale Abstand zwischen dem Sitzteil (2) einerseits und dem Stützteil (3) andererseits beliebig einstellbar ist, wobei die Teleskopstange (9) fest mit dem Sitzteil (2) einerseits und mit dem Stützteil (3) andererseits verbunden ist, wobei das Stützteil (3) und das Sitzteil (2) auf Rollen (8, 12) verfahrbar sind und wobei die Verbindung zwischen der Teleskopstange (9) und dem Sitzteil (2) einerseits und die Verbindung zwischen der Teleskopstange (9) und dem Stützteil (3) andererseits jeweils eine starre Verbindung ist.

2. Mobile Behandlungsstation nach Anspruch 1, deren Sitzteil (2) ein höhnver-stellbares Sitzelement (4) aufweist, das

3. Mobile Behandlungsstation nach einem der Ansprüche 1 bis 2, deren Stützteil (3) eine höhenverstellbare Auflagefläche (17) aufweist.

4. Mobile Behandlungsstation nach einem der Ansprüche 1 bis 3, an deren Stütz-teil (3) ein für die Aufnahme von Behandlungsutensilien geeigneter Aufnahmekorb (14), vorzugsweise verstellbar, angebracht ist.

5. Mobile Behandlungsstation nach einem der Ansprüche 1 bis 4, die am Stützteil (3) und/oder am Sitzteil (2) eine Bremsvorrichtung (13) aufweist.

## Claims

1. Mobile treatment station for use in the health sector, e.g. in hospitals, care homes for the elderly, rehabilitation centres and the like, with a seat part (2) on which a person providing treatment can sit, a support part (3) on which a limb of a person to be treated can be supported, and a mechanical connecting member (9) by means of which the seat part (2) and the support part (3) are connected to each other, **characterized in that** the mechanical connecting member (9) is adjustable in length and designed as a telescopic rod (9), such that the horizontal distance between the seat part (2) and the support part (3) can be adjusted as required, wherein the telescopic rod (9) is connected fixedly to the seat part (2) and to the support part (3), wherein the support part (3) and the seat part (2) are movable on rollers (8, 12), and wherein the connection between the telescopic rod (9) and the seat part (2) and the connection between the telescopic rod (9) and the support part (3) are in each case a rigid connectian.

2. Mobile treatment station according to Claim 1, of which the seat part (2) has a height-adjustable seat element (4).

3. Mobile treatment station according to one of Claims 1 and 2, of which the support part (3) has a height-adjustable bearing surface (17).

4. Mobile treatment station according to one of Claims 1 to 3, in which a retaining basket (14), suitable for accommodating treatment implements, is mounted, preferably adjustably, on the support part (3).

5. Mobile treatment station according to one of Claims 1 to 4, having a brake device (13) on the support part (3) and/or on the seat part (2).

## Revendications

1. Station de traitement mobile pour une utilisation dans des services de santé, par exemple dans des hôpitaux, des institutions pour personnes âgées, des centres de réhabilitation et analogues, avec une partie de siège (2), sur laquelle une personne traitée peut s'asseoir, une partie d'appui (3) sur laquelle un membre d'une personne à traiter peut s'appuyer, et un organe de liaison mécanique (9), au moyen duquel la partie de siège (2) et la partie d'appui (3) sont reliées l'une à l'autre, **caractérisée en ce que** l'organe de liaison mécanique (9) est réglable en longueur et est réalisé sous la forme d'une tige télescopique (9), de telle manière que la distance horizontale entre la partie de siège (2) d'une part et la partie d'appui (3) d'autre part puisse être réglée à volonté, dans laquelle la tige télescopique (9) est solidement attachée à la partie de siège (2) d'une part et à la partie d'appui (3) d'autre part, dans laquelle la partie d'appui (3) et la partie de siège (2) sont déplaçables sur des roulettes (8, 12) et dans laquelle la liaison entre la tige télescopique (9) et la partie de siège (2) d'une part et la liaison entre la tige télescopique (9) et la partie d'appui (3) d'autre part est chaque fois une liaison rigide.

2. Station de traitement mobile selon la revendication 1, dont la partie de siège (2) présente un élément de siège (4) réglable eh hauteur.

3. Station de traitement mobile selon l'une des revendications 1 à 2, dont la partie d'appui (3) présente une surface d'appui (17) réglable en hauteur.

4. Station de traitement mobile selon l'une quelconque des revendications 1 à 3, sur la partie d'appui (3) de laquelle est installé un panier de réception (14), de préférence réglable, convenant pour recevoir des ustensiles de traitement.

5. Station de traitement mobile selon l'une quelconque des revendications 1 à 4, qui présente un dispositif de freinage (13) sur la partie d'appui (3) et/ou sur la partie de siège (2).
